# EUROPÄISCHE PATENTSCHRIFT

(11) **EP 1 543 127 B1**
(45) Veröffentlichungstag und Bekanntmachung des Hinweises auf die Patenterteilung: **25.03.2009**
(21) Anmeldenummer: 03773452.2
(22) Anmeldetag: 21.09.2003
(51) Int. Cl.: C12N 15/57, C12N 9/50, A61K 38/48

(54) **FÜR PROTEINSPALTENDE ENZYME IN FORM VON SPEZIFISCHEN PROTEASEN KODIERENDE NUKLEINSÄURESEQUENZEN, ZUGEHÖRIGE POLYPEPTIDE UND VERWENDUNG VON ALLEN**
NUCLEIC ACID SEQUENCES CODING FOR PROTEOLYTIC ENZYMES IN THE FORM OF SPECIFIC PROTEASES, CORRESPONDING POLYPEPTIDES AND USE OF THE SAME
SEQUENCES D'ACIDE NUCLEIQUE CODANT POUR DES ENZYMES DE COUPURE PROTEIQUE SE PRESENTANT SOUS LA FORME DE PROTEASES SPECIFIQUES, POLYPEPTIDES CORRESPONDANTS, ET LEUR UTILISATION

(30) Priorität: 22.09.2002 DE 10244842
(43) Veröffentlichungstag der Anmeldung: 22.06.2005
(73) Patentinhaber: Stiftung Alfred-Wegener-Insitut für Polar- und Meeresforschung, 27570 Bremerhaven (DE)
(72) Erfinder: MOCK, Thomas, Seatle, WA 98 105 (US); VALENTIN, Klaus, 27580 Bremerhaven (DE)
(86) Internationale Anmeldenummer: PCT/DE2003/003180
(87) Internationale Veröffentlichungsnummer: WO 2004/029253

(56) Entgegenhaltungen:
- WO-A-02/02775
- DATABASE EMBL [Online] 18. September 2002 (2002-09-18), XP002287494 Database accession no. AY074514
- HUANG YUANHUI ET AL: "The calpain family and human disease" August 2001 (2001-08), TRENDS IN MOLECULAR MEDICINE, VOL. 7, NR. 8, PAGE(S) 355-362 , XP002287493 ISSN: 1471-4914 in der Anmeldung erwähnt das ganze Dokument
- PERRIN B J ET AL: "Calpain" INTERNATIONAL JOURNAL OF BIOCHEMISTRY AND CELL BIOLOGY, Bd. 34, Nr. 7, Juli 2002 (2002-07), Seiten 722-725, XP002287492 ISSN: 1357-2725
- ESKE, KRISTIN: "Zahllose Geheimnisse der Natur warten noch unter Wasser" BIOLOG, Nr. 3, 2000, Seiten 1-7, XP002263051 GREIFSWALD, ROSTOCK in der Anmeldung erwähnt
- "Leben unter extremen Bedingungen"[Online] 31. Januar 2002 (2002-01-31), XP002287495 Gefunden im Internet: URL:http://www.innovations-report.de/html/ berichte/geowissenschaften/bericht-7421.ht ml>
- DATABASE EMBL [Online] 15. Januar 2001 (2001-01-15), XP002287496 Database accession no. AB028639
- DATABASE EMBL [Online] 22. Juli 2003 (2003-07-22), XP002287500 Database accession no. CF076121

## Beschreibung

Die Erfindung bezieht sich auf eine für proteinspaltende Enzyme in Form von speziellen Proteasen kodierende Nukleinsäuresequenz, auf das zugehörige Polypeptid und auf Verwendungen sowohl der Nukleinsäuresequenz selbst als auch des zugehörigen Polypeptids.

Proteasen (auch: Proteinasen) sind eiweißspaltende Enzyme ("proteolytische Hydrolasen") die nach ihren Wirkungsmechanismen unterschieden werden. Eiweiße sind typisch gefaltete Polypeptid ketten. Die Proteasen werden daher auch als Peptidasen bezeichnet und in Endo- und Exopeptidasen unterschieden. Endopeptidasen spalten die Peptidketten im Inneren auf und erzeugen so kleinere Teilstücke, Exopeptidasen können die endständigen Aminosäuren abspalten. Proteolytisch wirkende Enzyme sind in biologischen Organismen allgegenwärtig und erfüllen typspezifische Zwecke. Sie können daher einerseits funktionsbezogen zu Therapiezwecken eingesetzt werden oder technischen Reinigungszwecken gegen proteinhaltige Verschmutzungen dienen. Die Familie der Calpain-Proteasen (CAPN)) ist ein wichtiger Vertreter der Proteasen und Gegenstand intensiver Forschungen.

### Calpain-7-Protease:

Bisher sind ca. 15 verschiedene CAPN bekannt. Die am weitesten erforschten CAPN bestehen aus einer großen 80kDa (kilo Dalton) - und einer kleinen 30kDa - Untereinheit. Die große Untereinheit besteht ihrerseits aus vier und die kleine Untereinheit aus zwei funktionalen Domänen. Die Domäne IV der großen und die Domäne VI der kleinen Untereinheit sind regelmäßig über ihre Faltungsformen ("EF-hands") als calziumbindende Domäne ausgeprägt. Es wird vermutet, dass die proteolytische Aktivität dieser CAPN erst durch die gebundenen Calziumatome ausgelöst wird. Die neueren CAPN (5,6,7,10,13; vergleiche z.B. **Veröffentlichung I)** verfügen dagegen in der Domäne IV der großen Untereinheit über keine solchen, calziumbindenden Strukturen. Grundsätzlich sind alle Mitglieder der CAPN-Familie gewebsspezifisch ausgebildet und erfüllen so die unterschiedlichsten Aufgaben.

CAPN nehmen oft Schlüsselstellungen in Stoffwechselwegen ein und sind am Verlauf von Krankheiten beteiligt. Z.B. sind die CAPN 1 und 2 Komponenten der Reaktionskaskade der Apoptose ("programmierter Zelltod") und damit am Verlauf der Alzheimer-Krankheit beteiligt. Auch auf Krebserkrankungen können CAPN Einfluss nehmen. Dabei werden z.B. Brust- und Darmkrebs durch die Erhöhung der zellulären Konzentration des p53-Markerproteins ausgelöst. Der Konzentrationsanstieg wird durch die Inhibierung von beteiligten CAPN erreicht, die die p53-Konzentration in gesunden Zellen normalerweise sehr gering halten (vergleiche **Veröffentlichung I**: "The calpain family and human disease", Yuanhui Huang und Kevin K.W.Wang in Trends in Molecular Medicine Vol.7 No.8 August 2001, pp 355-362, XP002287493).

Calpaine spielen eine besondere Rolle bei der Zell-Migration in der extrazellulären Matrix. Zellen wandern, indem sie sich am hinteren Ende auflösen und am vorderen Ende aufgebaut werden. Calpaine sind dabei durch proteolytische Spaltung von Proteinkomplexen des Stützapparats am Ende der Zelle an dieser Wanderungsbewegung beteiligt (vergleiche **Veröffentlichung II:** "V-SRC's hold over actin and cell adhesions", Frame, Fincham, Carragher, Eyke in Nature Reviews, Molecular Cell Biology, Vol.3, April 2002,pp 233-245).

Die beschriebenen Erkenntnisse wurden durch Untersuchungen an Wirbellosen, Säugetieren und dem Menschen gewonnen. CAPN in Pflanzen und Pilzen sind bisher nur durch Genom- und EST-Projekte identifiziert worden. Bei Pflanzen wird eine Wirkung in der Pathogenabwehr, bei Pilzen bei der Anpassung an alkalische Lebensbedingungen vermutet. Bei der gesamten Klasse der Kieselalgen sind CAPN bisher nicht bekannt.

Enzyme aus der Familie der Calpaine (jedoch nicht Calpain-7) werden in der Patentliteratur z.B. in EP1214427**,** CA2321270**,** DE10031932 **und** WO 02/02775 A sowie in **DATABASE EMBL** [Online], 18.September 2002, XP002287494, Database accession no. AY074514 offenbart. In Bezug auf die vorliegende Erfindung handelt es sich bei diesen Veröffentlichungen um technisch-wissenschaftlichen Hintergrund. Die WO 02/02775 A ist der nächste Stand der Technik für die vorliegende Erfindung und offenbart die Klonierung und Verwendung einer Calpain-12-Protease der Maus.

Aus der **Veröffentlichung III** "Zahllose Geheimnisse der Natur" von K. Eske (BIOLOG, Nr.3, 2000, Seiten 1-7, XP002263051 Greifswald, Rostock) ist es bekannt, kälteangepasste Enzyme aus Bakterien, die in Tiefseeregionen vorkommen, zu isolieren. Neben dem Vorteil, dass kälteangepasste Enzyme zur Expression keine erhöhten Temperaturen benötigen, ergibt sich bei den entsprechenden Organismen aus den Tiefseeregionen ein großes Vorkommenspotential, da 80% des die Erdoberfläche bedeckenden Wassers eine Temperatur unter 5 °C aufweist. Die Temperatur des Aktivitätsmaximums dieser Organismen und ihrer funktionellen Bestandteile liegt deutlich unter dem anderer Organismen aus temperierten und tropischen Breiten. Durch den Einsatz von kälteangepassten Mikroorganismen, auch in gentechnisch modifizierter Form, ist damit eine Produktion unter niedrigen Temperaturen mit einem gegenüber den herkömmlichen Gewinnungsverfahren mit nicht kälteangepassten Organismen deutlich geringerem Energieeinsatz entscheidend wirtschaftlicher als bei deren sonst üblichen Aktivitätstemperaturen.

Vor dem Hintergrund dieser bedeutenden Erkenntnisse und der WO 02/02775 A ist es daher die **Aufgabe** für die vorliegende Erfindung, zur wirtschaftlichen Produktion der erfindungsgemäßen Proteasen einen kälteangepassten Organismus zu finden, der Nukleinsäuresequenzen aufweist, die für kälteangepasste Enzyme in Form einer Calpain-Protease bei niedrigen Prozesstemperaturen (um 0°C) kodieren. Die erfindungsgemäße Lösung hierfür ist dem Anspruch 1 zu entnehmen. Es wird eine Nukleinsäuresequenz aus der kälteangepassten Diatomee *Fragilariopsis cylindrus* beansprucht, die für eine Calpain-7-Protease kodiert. Vorteilhafte Weiterbildungen und Verwendungen, die sich auch auf das zu der beanspruchten Nukleinsäuresequenz zugehörige Polypeptid beziehen, sind den unter- und nebengeordneten Ansprüchen zu entnehmen.

In **DATABASE EMBL** wird eine Calpain-1-Protease aus *Arabidopsis thaliana* offenbart. Die genannte DNA-Sequenz umfasst einen Abschnitt von acht Nukleotiden (1124-1131), deren Abfolge mit der eines Abschnitts (36-43) der beanspruchten SEQ No. 1 identisch ist. Eine gapfreie Übereinstimmung von 25 Nukleotiden in einer Folge von 26 Nukleotiden ergibt sich für *Herdmania curvata* und *Arachis hypogea arachin*. Eine längere gapfreie Übereinstimmung mit nur einer Identitätsabweichung konnte im Stand der Technik nicht ermittelt werden.

Mit der erfindungsgemäßen Lösung werden die an die Erfindung gestellten Anforderungen optimal erfüllt. Zum einen weist der die beanspruchte Nukleinsäuresequenz aufweisende Organismus spezifische Proteasen in Form eines Calpain-7-Protease-Enzyms auf, zum anderen entstammt der Organismus der antarktischen See, sodass dieses Enzym zusätzlich kälteangepasst ist und zu seiner Aktivität keine Wärmezufuhr benötigt wird. Die Kenntnis eines solchen, die beanspruchte Nukleinsäuresequenze enthaltenden Gens ist von elementarer Wichtigkeit, wenn es z.B. um die Bereitstellung großer Mengen solcher Nukleinsäuresequenzen bei geringem Energieaufwand geht. Die mikrobielle Synthese dieser Stoffe erzielt mit der speziellen, schnell wachsenden kälteangepassten Kieselalge *Fragilariopsis cylindrus* unter niedrigen Temperaturen hohe Erträge.

Durchgeführte Sequenzanalysen bestätigen, dass die erfindungsgemäß beanspruchte Nukleinsäuresequenze für eine Calpain-7-Protease kodiert. Durch die heutigen, entwickelten Methoden der automatisierten Sequenzierung von Nukleinsäureabschnitten ist es möglich geworden, in überschaubaren Zeiträumen gezielt Gene mit gewünschten Eigenschaften aus aussichtsreichen Organismen zu isolieren. Umfangreiche öffentliche Datenbanken mit bereits bestimmten, bekannten Funktionen zugeordneten Sequenzen dienen der schnelleren Verifizierung der Ergebnisse der mühsamen Suche. Zur Bereitstellung des für die Sequenzierung aufbereiteten Grundmaterials aus der speziellen Kieselalge dient eine Reihe von an sich bekannten Arbeitsschritten:

### 1) Isolation und Kultivierung des Organismus Fragilariopsis cylindrus

**Isolation :** Während einer Antarktisfahrt mit dem deutschen Forschungseisbrecher "Polarstern" in die Weddell-See wurde die Kieselalge *Fragilariopsis cylindrus* aus dem Meereis isoliert. Die Artbestimmung erfolgte in einfacher Weise durch die Typisierung der Struktur der Schale (vergleiche **Veröffentlichung VI** von Medlin & Priddle : "Polar marine diatoms", 2nd edition, British Antarctic Survey, Cambridge 1990, pp 182, 192).

**Kultivierung :** Die Kieselalge wurde bei 0°C in einem nährsalzangereicherten Medium 2 x f/2 bei 10µmol Photonen m⁻²s⁻¹ mit 24h Licht, gehältert (vergleiche: **Veröffentlichung VII** von Guillard & Ryther : "Studies of marine plancton diatoms, I.Cyclotella nana (Husted) and Detonula confervacea (Cleve)", 1962, Can.J.Microbiol. 8, pp 229:239). Für eine gesteigerte Expression der für die Kälteanpassung der Art verantwortlichen Gene wurden die Algen in der Mitte der exponentiellen Wachstumsphase auf -2°C abgekühlt. Dies entspricht dem Gefrierpunkt von Meerwasser. Nach 5 Tagen wurden die Boten-RNA (mRNA, messenger RNA) aus den Algen isoliert. Die mRNA repräsentieren alle gerade aktiven Gene, also auch diejenigen, die für die Kälteanpassung verantwortlich sind.

### 2) Isolation der mRNA

Die gesamte RNA wurde mit dem RNAeasy Plant Mini Kit (Firma Qiagen) isoliert. Aus ca. 100 µg RNA konnte mit Hilfe des Oligotex mRNA Midi Kit (Firma Qiagen) ca. 800 ng RNA für die cDNA-Synthese isoliert werden.

### 3) Herstellung und Screening einer cDNA-Bank

Die cDNA-Bank wurde auf der Basis der mRNA mit dem SMART cDNA-Library Construction Kit (Firma Clontech) hergestellt:
A) Von der mRNA wurde dazu mit Hilfe von Oligonukleotiden und CDS III/3' Primern der erste cDNA-Strang synthetisiert.
B) Anschließend wurde die Doppelstrangsynthese mit Hilfe der LD-PCR (long distance polymerase chain reaction) im Eppendorf Thermocycler unter folgendem Programm realisiert:
C)
   1. 5 min Denaturierung bei 95°C, anschließend 20 Zyklen von 6 min bei 68°C und 2 min bei 95°C.
   2. Nach einem SFil-Verdau (mit Restriktionsenzym aus Streptomyces fimbriatus) der cDNa wurde sie in CHROMA Spin-400 Säulen der Größe nach fraktioniert, so dass nur cDNAs der Länge >400bp (Basenpaare) für die Klonierung zum Einsatz kamen.
   3. Diese cDNAs wurden über Nacht bei 16°C in TripIEX2 Vektoren ligiert, die von λ-Phagen aufgenommen werden konnten. Der Titer dieser cDNA-Bank lag bei 2.7 x 10⁹ pfu/ml (plaque forming units / ml).
   4. Ein Blau-Weiß-Screening mit IPTG (Isopropyl-b-D-Thiogalactosid) und X-Gal (X-Galactosid) zeigte eine Rekombinationseffizienz von 70%.

### 4) Sequenzanalyse

Positive Phagen-Plaques wurden vom 5'-Ende mit λ-Primern vom Qiagen-Sequencing-Service sequenziert. Die Sequenzen wurden in der Genbank bei NCBI (National Center for Biotechnology Information) mit der BLASTX-Option auf ihre Homologien zu vorhandenen Sequenzen untersucht (BLAST-Protokoll vom 14.12.2001, Calpain-7-Protease; BLAST-Protokoll vom 24.04.2002). Für die Calpain-7-Protease konnte aus ca. 400 Sequenzen eine homologe Sequenz gefunden werden.

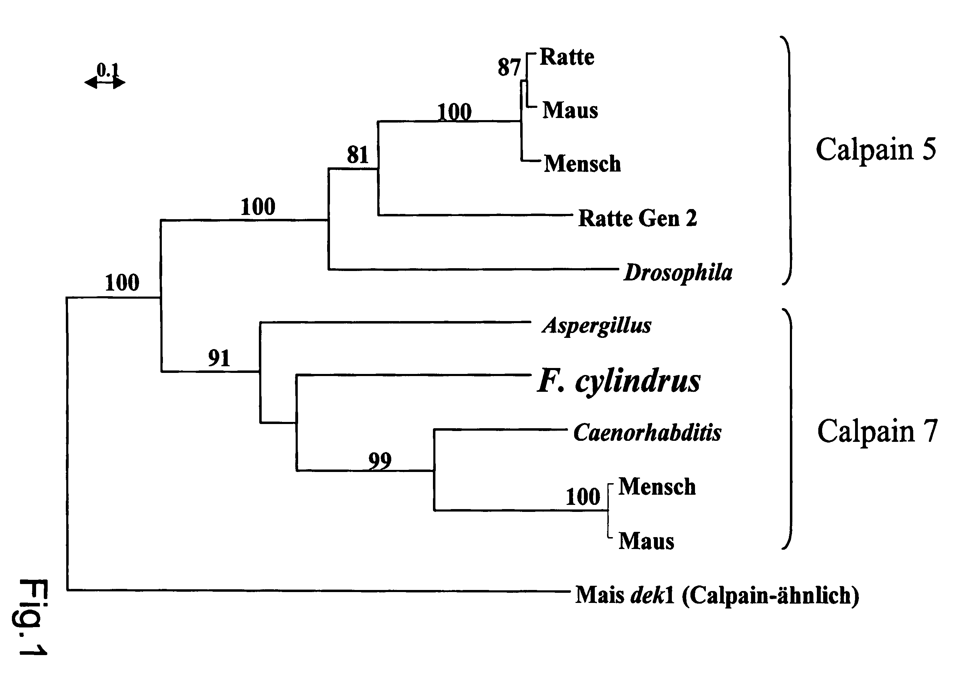

In **Figur 1** ist das Ergebnis einer phylogenetischen Analyse für die Calpain-7-Protease aus der Diatomee *Fragilariopsis cylindrus* dargestellt. Sie gruppiert mit signifikantem bootstrap support (allgemein bekannte mathematischstatistische Methode) von 99% mit anderen Calpain-7-Proteasen.

Es handelt sich daher bei dem kälteangepassten Enzym, das von den beanspruchten Nukleinsäuresequenzen nach der Erfindung aus der Diatomee *Fragilariopsis cylindrus* kodiert wird, mit einer Sicherheit von 99% ebenfalls um eine Calpain-7-Protease.

## Patentansprüche

1. Für proteinspaltende Enzyme in Form von speziellen Proteasen kodierende Nukleinsäuresequenz,
**dadurch gekennzeichnet, dass**
die Nukleinsäuresequenz aus der kälteangepassten marinen Diatomee *Fragilariopsis cylindrus* stammt und für eine Calpain-7-Protease gemäß SEQ ID No.1 kodiert

2. Nukleinsäuresequenz nach Anspruch 1,
**dadurch gekennzeichnet, dass**
die Nukleinsäuresequenz als DNA oder RNA, vorzugsweise als doppelsträngige DNA ausgebildet ist.

3. Nukleinsäuresequenz nach Anspruch 1 oder 2
**dadurch gekennzeichnet, dass**
die Nukleinsäuresequenz in Vektoren, vorzugsweise in Expressionsvektoren enthalten ist.

4. Verwendung der Nukleinsäuresequenz nach Anspruch 3 zur Expression oder Überexpression des Enzyms Calpain-7-Protease in Wirtsorganismen.

5. Zu den Nukleinsäuresequenzen gemäß Anspruch 1 oder 2 gehöriges Polypeptid, das aus der mit der Nukleinsäuresequenz gemäß SEQ ID No.1 kodierten Aminosäuresequenz besteht.

6. Verwendung des Polypeptids nach Anspruch 5 zu Reinigungszwecken bei proteinhaltigen Verschmutzungen.

## Claims

1. A nucleic acid sequence encoding for protein-splitting enzymes in the form of special proteases,
**characterised in that**
said nucleic acid sequence originates from the cold-adapted marine diatom *Fragilariopsis cylindrus* and encodes for a calpain 7 protease according to SEQ 10 No. 1.

2. The nucleic acid sequence according to claim 1,
**characterised in that**
said nucleic acid sequence is formed as DNA or RNA, preferably as double-stranded DNA.

3. The nucleic acid sequence according to claims 1 or 2,
**characterised in that**
said nucleic acid sequence is contained in vectors, preferably in expression vectors.

4. A use of the nucleic acid sequence according to claim 3 for expressing or over-expressing said enzyme calpain 7 protease in host organisms.

5. A polypeptide associated with the nucleic acid sequences according to claims 1 or 2, consisting of the amino acid sequence encoded with said nucleic acid sequence according to SEQ ID No. 1.

6. A use of the polypeptide according to claim 5 for cleaning purposes for protein-containing contaminations.

## Revendications

1. Séquence nucléotidique codant pour des enzymes protéolytiques sous forme de protéases particulières,
**caractérisée en ce que**
ladite séquence nucléotidique est issue la diatomée marine psychrotolérante *Fragilariopsis cylindrus* et code pour une protéase calpaïne-7 selon la SEQ ID n° 1.

2. Séquence nucléotidique selon la revendication 1,
**caractérisée en ce que**
ladite séquence nucléotidique se présente sous forme d'ADN ou d'ARN, s'agissant de préférence d'un ADN double brin.

3. Séquence nucléotidique selon les revendications 1 ou 2,
**caractérisée en ce que**
ladite séquence nucléotidique est contenue dans des vecteurs, de préférence dans des vecteurs d'expression.

4. Utilisation de la séquence nucléotidique selon la revendication 3 pour exprimer ou surexprimer l'enzyme protéase calpaïne-7 dans des organismes hôtes.

5. Polypeptide relatif aux séquences nucléotidiques selon les revendications 1 ou 2, lequel est constitué de la séquence d'acides aminés codée par la séquence nucléotidique selon la SEQ ID n° 1.

6. Utilisation du polypeptide selon la revendication 5 pour enlever des salissures contenant des protéines.


### Sequenzprotokoll

<110>Stiftung Alfred-Wegener-Institut für Polar- und Meeresforschung, Bremerhaven
<120>Für eine Calpain-Protease kodierende neue Nukleinsäuresequenz aus einer kälteangepassten marinen Diatomee Fragilariopsis cylindrus
<130>AWI 01/0902 DE
<160>2
<210>1
   <211>544
   <212>DNA
   <213>Fragilariopsis cylindrus
<400>1
   GG GAA TTC GGC CTT ACG GCC GGG GAT GAT GGA ATG TTC TGG ATT AGT      47
      Glu Phe Gly Leu Thr Ala Gly Asp Asp Gly Met Phe Trp Ile Ser
       1               5                  10                  15
   TGG GAG GAT GTC TTG CTT TAT TTC CGC AAT TTA CAA TTA TCA TGG AAT     95
   Trp Glu Asp Val Leu Leu Tyr Phe Arg Asn Leu Gln Leu Ser Trp Asn
                   20                  25                  30
   CCC AAA CTA TTT GCG TAT CGG ATG ACT ACT CAT GGC TTA TGG CCA AAG    143
   Pro Lys Leu Phe Ala Tyr Arg Met Thr Thr His Gly Leu Trp Pro Lys
               35                  40                  45
   GAT CAG GGA CCA CAA AAT GAT GCA TTT AAT GTC GGA GAG AAT CCA CAA    191
   Asp Gln Gly Pro Gln Asn Asp Ala Phe Asn Val Gly Glu Asn Pro Gln
           50                  55                  60
   TAT ATC ATG TCT TTC TCC GAA AAA GCT GTA TCG AGT AAA CCA ACG ATT   239
   Tyr Ile Met Ser Phe Ser Glu Lys Ala Val Ser Ser Lys Pro Thr Ile
       65                  70                  75
   TGG GTA CTG ATA TCA AGG CAT GTA AGC AAA CAG GAG CAA GAA GGT GCT    287
   Trp Val Leu Ile Ser Arg His Val Ser Lys Gln Glu Gln Glu Gly Ala
   80                  85                  90                  95
   GAG GTG AAT GAT TTC TTA ACC ATA CAT CTC GTT AGA AAC TCG GCT ACA    335
   Glu Val Asn Asp Phe Leu Thr Ile His Leu Val Arg Asn Ser Ala Thr
                   100                 105                 110
   TTA GAA AGA GTT TGG TAT CCC CAT GGA AAA GCA ACG ATT GCT AAT GGA    383
   Leu Glu Arg Val Trp Tyr Pro His Gly Lys Ala Thr Ile Ala Asn Gly
               115                 120                 125
   TGC TAT ACA AAC AAT CCA CAC GTG CTT TTA CGA TAC GAT GTT TCC GGA    431
   Cys Tyr Thr Asn Asn Pro His Val Leu Leu Arg Tyr Asp Val Ser Gly
           130                 135                 140
   CCT GAA GAT CAA TTT ATC TCG TTA GTA CTG TCT CAA CAC GAA AAA ACT    479
   Pro Glu Asp Gln Phe Ile Ser Leu Val Leu Ser Gln His Glu Lys Thr
       145                 150                 155
   CAA GAT CTA TCA TAC ACT CTC TCT TGT TAC TGT ACC GAA CCC TTT ACA    527
   Gln Asp Leu Ser Tyr Thr Leu Ser Cys Tyr Cys Thr Glu Pro Phe Thr
   160                 165                 170                 175
   CTA GGA AGA CCA CCA AA                                             544
   Leu Gly Arg Pro Pro
                   180
<210>2
   <211>544
   <212>DNA
   <213>Fragilariopsis cylindrus
<400>2
   TCA AAC GAT GGT GCG CAA TAC GTA GTA GAG AAA TCG ATA CTG GTA GGT    48
   Ser Asn Asp Gly Ala Gln Tyr Val Val Glu Lys Ser Ile Leu Val Gly
    1               5                  10                  15
   TCA GTG AAT TAT CCT GTA AAA GAT CCA TTT AAT CAG ATG AAA CGT GGA    96
   Ser Val Asn Tyr Pro Val Lys Asp Pro Phe Asn Gln Met Lys Arg Gly
               20                  25                  30
   TCA CTT CAA ACC TAC TCA GAT TCA TGG ACC GAA CGG GAT CGT ACC TCA   144
   Ser Leu Gln Thr Tyr Ser Asp Ser Trp Thr Glu Arg Asp Arg Thr Ser
           35                  40                  45
   TTT GTC ATG GCA TCA CGT AAC TTA GCC GAT TTT CGT AAT AAC GTG AAG   192
   Phe Val Met Ala Ser Arg Asn Leu Ala Asp Phe Arg Asn Asn Val Lys
       50                  55                  60
   GTA ACG ATC GAT GCT GTT TTT AAT CCA CTT TTT ATC AAC GAG GAA TAC   240
   Val Thr Ile Asp Ala Val Phe Asn Pro Leu Phe Ile Asn Glu Glu Tyr
   65                  70                  75                  80
   AAA TGG ATC TTT CGT CAA GAA GGC TGG AGG TTA GAG ACA CCT GAC AAT   288
   Lys Trp Ile Phe Arg Gln Glu Gly Trp Arg Leu Glu Thr Pro Asp Asn
                   85                  90                  95
   GTC AAC CTA CTT ATC AAT GGG AAC GCT TAT GTA AAC GCT AAG GCC GAC   336
   Val Asn Leu Leu Ile Asn Gly Asn Ala Tyr Val Asn Ala Lys Ala Asp
               100                 105                 110
   CAG ATG GAC CCC CAA GAG GTT ATG ATA AAG CAA ATC TAC AGC AAT CTC   384
   Gln Met Asp Pro Gln Glu Val Met Ile Lys Gln Ile Tyr Ser Asn Leu
           115                 120                 125
   TTT GCT GAT CAC GTG TAT AGC AAA AGT CCA AAA GGA GAC GCC GCC CAA   432
   Phe Ala Asp His Val Tyr Ser Lys Ser Pro Lys Gly Asp Ala Ala Gln
       130                 135                 140
   GTA GTC ACC ATG ACA TTG GCA CCA AGG GCG AAT TCT GCA GAT ATC CAT   480
   Val Val Thr Met Thr Leu Ala Pro Arg Ala Asn Ser Ala Asp Ile His
   145                 150                 155                 160
   CAC ACT GGC GGC CGT CTC GAG CAT GCA TCT AGA GGG CCC AAT TCG CCC   528
   His Thr Gly Gly Arg Leu Glu His Ala Ser Arg Gly Pro Asn Ser Pro
                   165                 170                 175
   TAT AGT GAG TCG TAT T                                             544
   Tyr Ser Glu Ser Tyr
               180 181